# EUROPEAN PATENT APPLICATION

(11) **EP 1 160 570 A1**
(43) Date of publication of application: **05.12.2001**
(21) Application number: 00111444.6
(22) Date of filing: 27.05.2000
(51) Int. Cl.: G01N 33/15, G01N 33/50

(54) **Method for the identification of agents and genes influencing cardiovascular function**

(71) Applicant: ARTEMIS Pharmaceuticals GmbH, 51063 Köln (DE)
(72) Inventor: Langheinrich, Ulrike, 72072 Tübingen (DE)
(74) Representative: Helbing, Jörg, Dr. Dipl.-Chem.

(57) **Abstract**

The present invention relates to a method for the identification of agents influencing cardiovascular function utilizing teleost alevin. Said method is also applicable for the identification of genes regulating heart function.

## Description

The present invention relates to a method for the identification of agents influencing cardiovascular function utilizing teleost alevin. Said method is also applicable for the identification of genes regulating heart function.

### Summary of the Related Art

Conventional drug-finding techniques are time consuming and costly. Recently high-throughput *in vitro* screening methods became available allowing pharmaceutical testing of a great number of compounds within a short period of time (S.A. Sundberg, Curr. Opin. Biotechnol., 11(1):47-53 (2000); J. Kuhlmann, Int. J. Clin. Pharmacol. Ther., 37:575-583 (1999); J.R. Zysk et al., Comb. Chem. High Throughput Screen, 1(4) 171-183 (1998); B.A. Kenny et al., Prog. Drug Res., 51:245-269 (1998)).

The compounds identified by such screening methods are then tested in mammalian systems, such as mice or rats, which are of course far slower than *in vitro* testing methods. Recently teleosts were found to be effective animal models for mammals and humans. In particular, WO 99/42606 discloses a method for screening an agent for angiogenesis activity or cell death activity (viz. the agent to be tested is administered to a teleost (e.g., zebrafish) and a response in the teleost is detected); and U.S. Patent No. 5,565,187 discloses a method for studying capillary circulation on living salmonid or other teleosts with yolk sac attached (viz. a fluorescent dye complex and a tracer is injected into the yolk sac and the fluorescence is tracked in the systemic circulation).

However, there is still need for a reliable screening method for cardiovascular drugs.

### Summary of the Invention

Surprisingly it was found that the effect of cardiovascular medicaments on the heart beat of teleost alevin can be determined just by bathing teleost alevin in a drug-containing medium and subsequent visual inspection of the heart function, e.g., via a microscope. The present invention thus provides
(1) a method for the identification of agents influencing cardiovascular function which comprises
   (a) preparing a medium in which fertilized teleost alevin is bathed,
   (b) adding the agent to be tested to the medium with teleost alevin and
   (c) visually monitoring heart beat rate, rhythm of the heart beat, contractility of the heart and/or blood flow;
(2) a preferred embodiment of (1) above, in which prior step (b) or simultaneous with step (b) a pharmaceutically active agent, preferably having an adverse effect (relative to the desired effect of the agents to be tested) on heart beat rate, rhythm of the heart, contractility of the heart and/or blood flow, is added to the medium with teleost alevin; and
(3) a method as defined in (1) and (2) above, which is suitable for identification of genes involved in cardiovascular action.

The present invention is described in more detail below.

### Brief description of the Figures

Fig. 1 shows the effects of cardiac pharmaceuticals (at a concentration of 100 µM) on the heart beat rate of 2d old zebrafish larvae.

Fig. 2 shows the effect of increasing concentrations of the Ca²⁺ channel blockers nifedipine and nimodipine on the heart beat rate of zebrafish larvae (2d post fertilization).

Fig. 3 shows the reversibility of the nifedipine-induced decrease in heart beat rate of 4d old zebrafish larvae by simultaneous addition of the Ca²⁺ channel agonist Bay K 8644.

Fig. 4 shows the effect of the Bay K 8644 on the heart beat rate of 4d old zebrafish larvae.

Fig. 5 shows re-animation of 3 d old zebrafish larvae with nifedipine-induced heart arrest by addition of Bay K 8644.

Fig. 6 shows the reversibility of the nifedipine-induced decrease of heart contractility of 3d old zebrafish larvae.

### Detailed description of the Invention

The present invention provides a method by which new agents influencing cardiovascular function can be found.

"Fertilized teleost alevin" in accordance with the present invention means fertilized teleost eggs (hereinafter also shortly referred to as "teleost larvae"). "Teleosts" in accordance with the present invention include zebrafish and medaka. The preferred teleost is zebrafish.

The preparation of a medium in which fertilized teleost alevin is bathed in accordance with steps (a) of embodiment (1) of the invention comprises the provision of a suitable medium, adding the teleost alevin (preferably zebrafish larvae), and incubating the teleost alevin, preferably for 2 to 7 days at 22 to 28°C. Suitable media for rasing teleost alevin are known in the art and include low salt, buffer solutions (e.g., solutions containing less than 10 mM salts (alkaline and earth alkaline salts) and less than 20 mM buffer substance). For zebrafish larvae the most preferred medium is the so-called "embryo medium" comprising:
4.9 mM NaCl, 170 µM KCI, 329 µM CaCl₂, 331 µM MgSO₄, pH 7.2 (M. Westerfield, The zebrafish book, University of Oregon Press, Eugene, OR, USA (1993)) which is supplemented with 10 mM HEPES.

In step (b) compounds to be tested are added to the medium prepared in step (a), preferably 2 to 5 d after fertilization of the teleost alevin. The compound to be tested is preferably added at concentration of 100 nM to 100 µM, most preferably at 1-10 µM. Thereafter, preferably immediately to 48 h after addition of the compound to be tested, heart beat (rate, rhythm and contractility) and blood flow of the teleost is visually monitored, e.g., via a microscope such as a dissecting microscope.

A main advantage of teleost (and especially of zebrafish) is that the larvae can be raised in a large number and that they are transparent facilitating the microscopic inspection of the heart beat. Due to the prominent location of the heart just beneath the skin, agents acting on the heart rapidly reach their target.
Responses to the cardiac pharmaceuticals tested are similar between mammals and fish. Thus, the teleosts are extremely well suited organisms for the study of compounds acting on heart function, e.g. by performing high-throughput screening (HTS) assays.

With methods of the present invention, teleosts can be used to screen a large number of compounds for their effects on heart beat. For example, using 24 well format and manual techniques (addition of the drug, pipetting of the larvae, microscopy) about 300 substances per day and person can be tested for their effects on the heart (2 concentrations per agent to be tested, each well containing about 10 zebrafish larvae). One particular advantage of the present invention, in comparison to cell-free or even-cell based conventional *in vitro* HTS assays, is that the agents tested act on an intact heart integrated in the whole-body physiology. In particular, drugs influencing heart beat rate, contractility, and blood flow could be found with the developed method.

The advantages in comparison to conventional drug-finding techniques are reliability, speed, and costs. Furthermore, hits arising from usual industrial high-throughput screening assays could be prioritized using teleosts in advance to experiments with mice or rats. Those hits might be substances with an expected effect on the heart function but also hits with other targets than the heart, in order to study potential side-effects (e.g., arrhythmia, bradycardia, tachycardia, cardiac failure) of these compounds.

In a preferred embodiment, for the simulation of diseases, and to thereby increase the responsiveness of teleosts to the agents applied (or even rendering them responsive), in step (b) - prior to or simultaneous with the addition of the agent to be tested - a pharmaceutically active agent (hereinafter "sensitizing agent") is added to the medium, for example Ca²⁺ channel blockers or agents causing arrhythmia, in order to find substances reverting the effects of the sensitizing agent.

The addition of Ca²⁺ channel blockers prior to or simultaneous with an agent-containing medium may be used to detect those agents which lead to an increase in contractility of the teleost heart (putative drugs for the treatment of congestive heart failure). Namely the heart beat rate, contractility and blood flow is lowered by addition of a Ca²⁺ channel blocker, like nifedipin. Putative anti-arrhythmic agents can be identified by studying heart beats of teleost larvae which were bathed prior to or simultaneous with the addition of the agents in a solution of an arrhythmia-inducing drug. Thereby human diseases can be simulated (e.g., congestive heart failure or arrhythmia). Thus, drug-treated teleost presents the first whole-animal disease model, which is suited for high-throughput screenings. Using high concentrations of nifedipine (e.g. 50 µM) an arrest of the heart beat can be induced in teleost larvae, thereby offering the possibility to screen for compounds which can reanimate the heart to beat, potential drugs for the treatment of cardiac infarction.

The method of the present invention, e.g., application of Ca²⁺ channel blockers to the teleost larvae, is moreover suitable for the identification of special genes involved in cardiovascular function. These genes are expected to encode especially those proteins, which will, after applying specific antagonists, lead to an increase in cardiac contractility. Up to now, no method exists, which could yield a similar number of new genes of this specific type. By performing high-throughput screening assays with these new proteins, antagonists (medicaments) for the treatment of congestive heart failure could be found.

In accordance with said embodiment of the present invention, teleost larvae, preferably zebrafish larvae carrying hetero- or homozygous mutations are sensitized by adding for example Ca²⁺ channel blocker (step (a) of the screening method). Such mutations can, e.g., be induced by ethylnitrosurea (P. Haffter et al., Development 1996, 123, 1-36) or insertion mutagenesis (A. Amsterdam et al., Genes Dev., 13(20):2713-24 (1999)).

By performing a dominant or recessive genetic screen in accordance with the method of the invention and by applying Ca²⁺ channel blockers to the teleost larvae carrying mutations, phenotypes which show resistance to the drug applied can be identified. This means, that the heart beat and blood flow is less influenced by the Ca²⁺ channel blocker than in wildtypes. It is believed that these phenotypes carry mutations in genes encoding proteins, which will, after applying specific antagonists, lead to an increase in cardiac contractility. The reason for this feature is, that the underlying mutations are quite likely "loss of function" mutations ("gain of function" mutations are quite rare). Thus, with this kind of screen new targets (for which antagonists could be developed) for the treatment of congestive heart failure can be found, e.g. after positional cloning of the mutations.

Genotypes with the above mentioned mutations do not show easily identifiable phenotypes if not previously incubated with, for example, Ca²⁺ channel blockers, and are likely to be overlooked in conventional genetic screens for the heart beat (zebrafish, medaka, mice screens), because contractility is near the optimum. This conclusion is supported by the observation that an increase in cytosolic calcium induced by the Ca ²⁺ channel agonist (+/-)Bay K 8644 (1,4-dihydrn-2,6-dimethyl-5-nitro-4-[2'-(trifluoromethyl) phenyl]-3-pyridinecarboxylic acid methyl ester) has nearly no effect on cardiac performance of untreated fish, in contrast to its effects on nifedipine-treated larvae. Therefore, for getting not only genotypes but also phenotypes in a genetic screen for the contractility, the induction of a "disease status", (lowering the contractility by addition of the Ca²⁺ channel blocker) in the organism prior to screening will be of great advantage.
Since the pharmaceutical industry is much more interested (due to a higher success rate) in targets for which antagonists rather than agonists have to be developed, the described invention represents a major further development of conventional screens for contractility, which would yield mainly targets for agonists (for the treatment of heart failure).

Loss of function mutations in genes, encoding proteins, which after inhibition, cause an increase in contractility, will probably also have no obvious phenotypes in humans. Thus, screening families with heart defects and also differential display techniques are not likely to reveal these interesting targets.

The invention is further explained by the following non-limitative examples.

### Examples

### Material and Methods

- zebrafish larvae
- embryo medium 4.9 mM NaCl, 170 µM KCI, 329 µM CaCl₂, 331 µM MgSO₄, 10 mM Hepes, pH 7.2 (Sigma, p.a. grade)
- DMSO (Sigma; ultrapure)
- MESAB solution (0.4 % ethyl-m-aminobenzoate methanesulfonate + 1 % Na₂HPO₄ x 2 H₂O, pH 7.2)
- Ca²⁺ channel inhibitor, for example nifedipine or nimodipine (Sigma)
- petri dishes (10 cm and 4 cm in diameter) (Greiner, Nürtingen)
- 50 ml plastic tubes (screwcaps) (Greiner, Nürtingen)
- dissecting microscope (MZ-FL III; Leica)
- incubator (Heraeus)
- timer

### Example 1

Zebrafish larvae were raised according to established protocols (M. Westerfield, The zebrafish book, University of Oregon Press, Eugene, OR, USA (1993)).
About 50 zebrafish larvae were incubated for 2-7 d at 22-28°C in petri dishes filled with 30 ml embryo medium.
At the day of the experiment 10 larvae were transferred to small petri dishes filled with 10 ml embryo medium, 0.5 ml MESAB solution and 10 µl of the respective drug (or + 10 µl of a second drug) added from a 1000-fold stock solution prepared with DMSO, these three components were previously mixed in 50 ml plastic tubes (controls only receive 10 µl DMSO).
At certain time points the contractility of the heart and blood flow was monitored, and the heart rate was determined by counting the heart beat with the aid of a stereo-microscope.

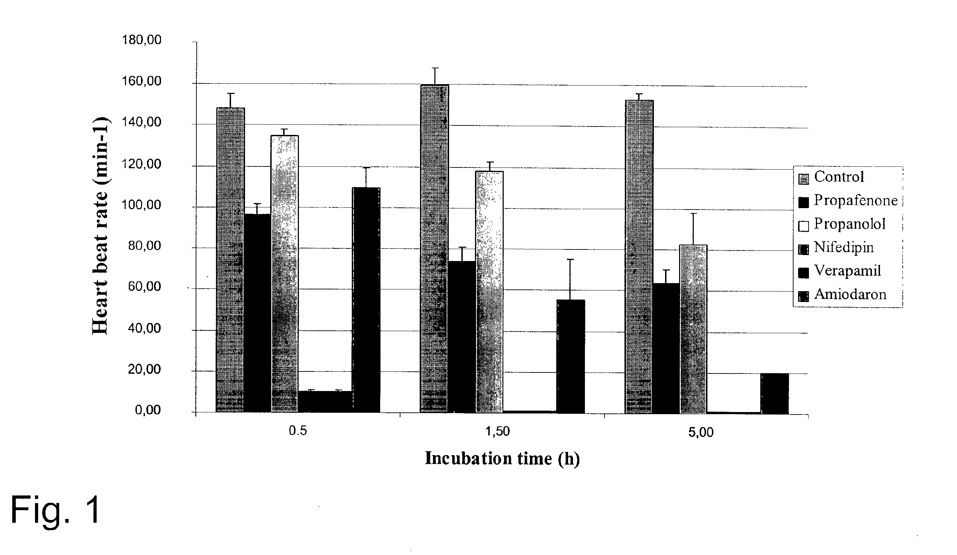

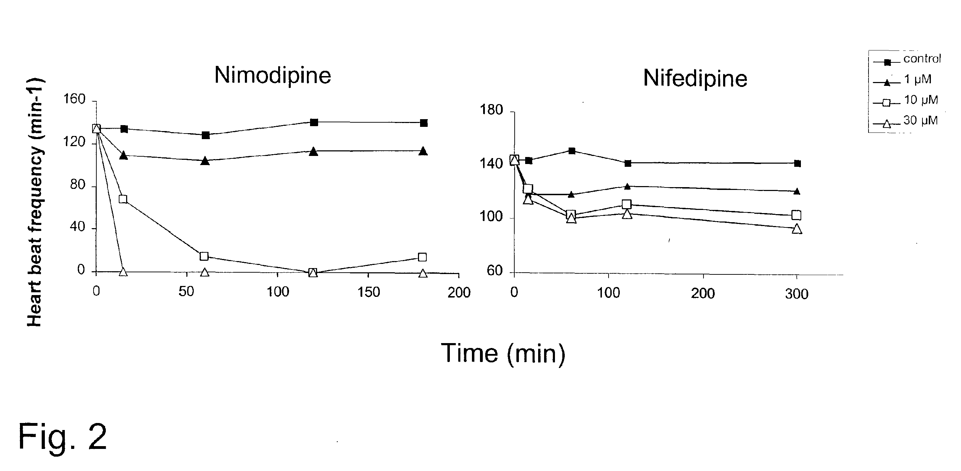

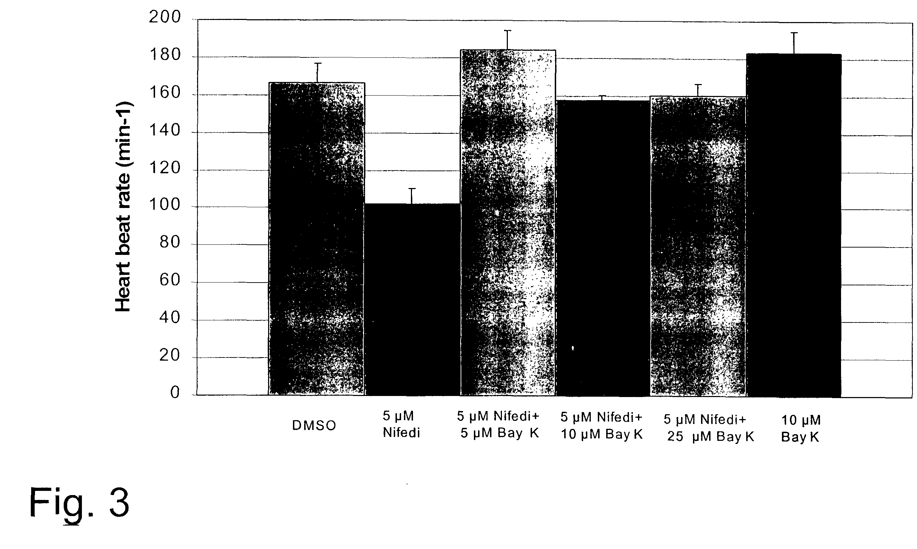

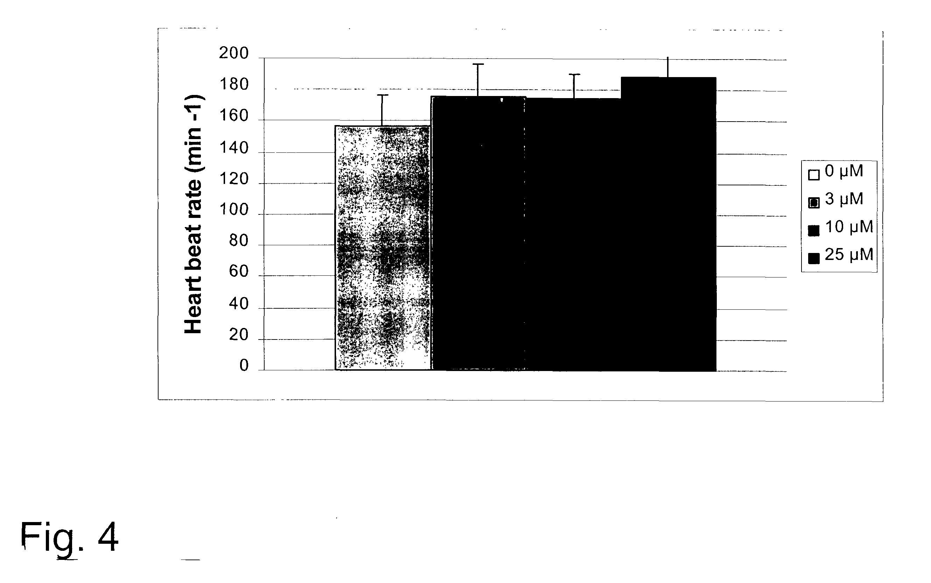

Several pharmaceuticals known to have an effect on heart beat rate and/or contractility in mammals were tested. The β-adrenergic blocker propanolol and the Ca²⁺ channel blockers verapamil (Fig. 1), nifedipine and nimodipine (Fig. 2), induced a decrease in heart beat rate, contractility and blood flow, similar to rodents and humans. The drugs propafenone (sodium channel blocker) and amiodarone (potassium channel blocker) have similar effects as the drugs mentioned above, indicating similarities between mammalian and teleost cardiac channels (Fig. 1). Bay K 8644, a Ca²⁺ channel agonist, acting on dihydropyridine-sensitive Ca ²⁺ channels in mammals, led to a strong increase in contractility of nifedipine-treated zebrafish, in contrast to untreated fish (Fig. 3 and 4). Thus, the zebrafish heart reacts in a similar way (also with respect to the concentrations of the drugs and reaction time) as isolated mammalian cardiomyocytes. This feature shows, that zebrafish with artificially reduced contractility can be used for screens aimed at finding agents increasing the contractility of the heart.

### Example 2

Zebrafish larvae of the F₃ or F₂ generation carrying heterozygous or homozygous mutations (induced by ethylnitrosurea or insertional mutagenesis) are raised according to the procedures described in Example 1. A genetic screen was performed by incubating about 25 zebrafish larvae of the F₂ (dominant screen) or F₃ (recessive screen) generation per crossing for 2-7 d at 22-28°C in petri dishes filled with 30 ml embryo medium. At the day of the experiment 25 larvae were incubated with 30 ml embryo medium, 1.5 ml MESAB solution and 30 µl of the respective drug added from a 1000-fold stock solution prepared with DMSO. At certain time points the contractility of the heart, heart rate and blood flow were monitored with the aid of a stereo-microscope. In the case 25 % of the larvae inspected show resistance to the drugs applied the parents will be outcrossed to another zebrafish line (WIK) and subsequently the mutations will be cloned, e.g. by positional cloning.

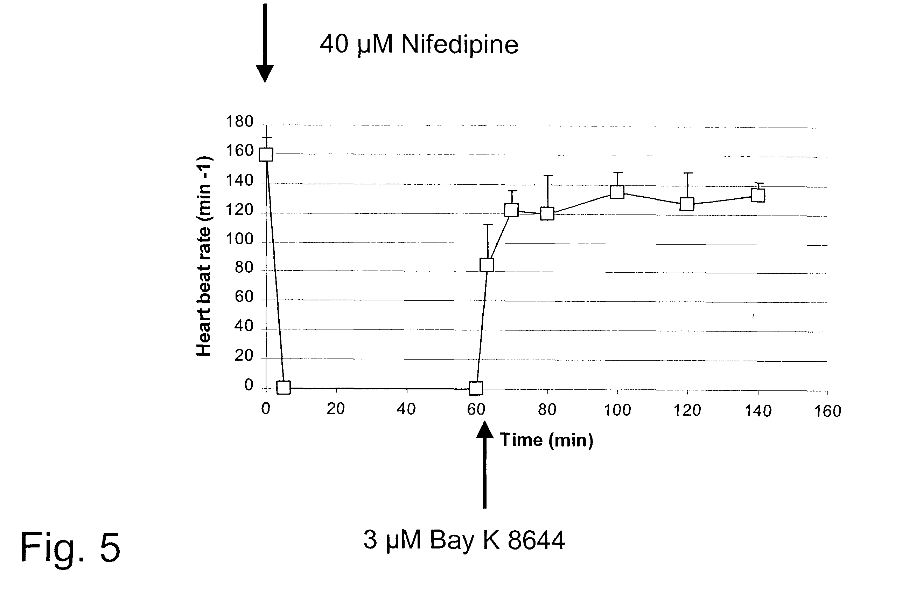

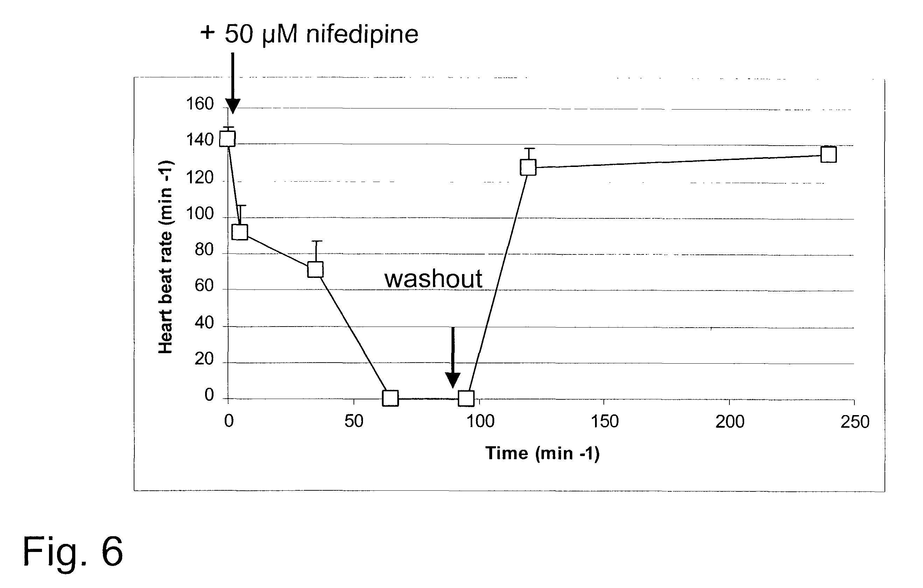

It was found that low concentrations of Ca²⁺ channel inhibitors (nifedipine, nimodipine) applied to the bathing medium induced a strong decrease in heart contractility, blood flow and heart beat rate of zebrafish larvae. Fig. 2 shows dose-response curves for nimodipine and nifedipine at 2d postfertilization. The effect was reversible after washout of the drug. Even zebrafish with an arrest of the heart beat for 30 min (induced by adding 50 µM nifedipine for 90 min) could be re-animated by washout of the drug, such that the heart beat rate and contractility were nearly the same as in controls (Fig. 6). The effects of nifedipine were completely blockable by the simultaneous addition of the Ca²⁺ channel agonist Bay K 8644, strongly indicating that the observed effects of nifedipine are specific and indeed the result of an inhibition of Ca²⁺ channels of the dihydropyridine type (Fig. 3). zebrafish larvae with an arrest of the heart beat (induced by adding 50 µM nifedipine for 1 h) can also be re-animated within minutes by addition of Bay K 8644 to the bathing solution (Fig. 5). Bay K 8644 applied alone to the surrounding medium, had nearly negligible effects on heart beat rate, contractility and blood flow (Fig. 4), indicating that a further increase in the cytosolic Ca²⁺ concentration has no consequence on these parameters.

## Claims

1. A method for the identification of agents influencing cardiovascular function which comprises
(a) preparing a medium in which fertilized teleost alevin is bathed;
(b) adding the agent to be tested to the medium with teleost alevin; and
(c) visually monitoring heart beat rate, rhythm of the heart beat, contractility of the heart and/or blood flow of the teleost alevin.

2. The method of claim 1, wherein the fertilized teleost alevin are zebrafish larvae.

3. The method of claim 1 or 2, wherein step (a) comprises incubating fertilized teleost alevin for about 2 to 7 days at about 22 to 28°C.

4. The method of claims 1 to 3, wherein the agent to be tested is added at a concentration of about 100 nM to about 100 µM, preferably about 1 to 10 µM.

5. The method of claims 1 to 4, wherein the heart beat rate, rhythm of the heart beat, contractility of the heart and/or blood flow are monitored with a microscope, preferably a dissecting microscope.

6. The method of claims 1 to 5, wherein prior to step (b) or simultaneous with step (b) a pharmaceutically active agent (sensitizing agent), preferably a sensitizing agent having an adverse effect (relative to the desired effect of the agent to be tested) on heart beat rate, blood flow and/or contractility in mammals, is added to the medium with zebrafish larvae.

7. The method of claim 6, wherein the sensitizing agent is added at a concentration of about 1 to 10 µM.

8. The method of claim 6 or 7, wherein the sensitizing agent is selected for example from β-adrenergic blockers, Ca²⁺ channel blockers, sodium channel blockers or potassium channel blockers.

9. The method of claims 1 to 8, which is suitable for the identification of genes involved in cardiovascular action.

10. The method of claim 9, wherein the genes encode proteins leading to an increase in cardiac contractility.

11. The method of claims 9 or 10, wherein the teleost alevin carries heterozygous or homozygous mutations.
